Europäisches Patentamt

European Patent Office

Office européen des brevets

(11) Publication number: **0 541 518 B1**

## EUROPEAN PATENT SPECIFICATION

(45) Date of publication of patent specification: **16.08.95**

(51) Int. Cl.⁶: **G01N 3/08**, G01N 3/20, G01N 33/34

(21) Application number: **93200177.9**

(22) Date of filing: **15.07.87**

(60) Publication number of the earlier application in accordance with Art.76 EPC: **0 253 644**

(54) System and method for determination of the strength of sheet materials.

(30) Priority: **18.07.86 US 887292**
**16.10.86 US 920107**

(43) Date of publication of application:
**12.05.93 Bulletin 93/19**

(45) Publication of the grant of the patent:
**16.08.95 Bulletin 95/33**

(84) Designated Contracting States:
**DE FR GB IT SE**

(56) References cited:
**EP-A- 0 200 650**
**CH-A- 475 609**
**DE-C- 751 614**
**US-A- 2 966 792**

**JOURNAL OF PHYSICS E. SCIENTIFIC IN-STRUMENTS vol. 4, no. 8, August 1971, Bristol, GB, pp. 615-618 ; MARTIN et al. : "A High Temperature Four-Point Bending Machine For Testing Thin Sheets Of Refractory Materials"**

(73) Proprietor: **MEASUREX CORPORATION**
**One Results Way**
**Cupertino, California 95014 (US)**

(72) Inventor: **Houghton, Paul Joseph**
**21050 Bear Creek Road**
**Los Gatos, CA (US)**
Inventor: **Anderson, Leonard M.**
**1355 Stockbridge Drive**
**San Jose, CA (US)**
Inventor: **Goss, John Douglas**
**6151 Hancock Avenue**
**San Jose, CA (US)**

(74) Representative: **Atkinson, Peter Birch et al**
**MARKS & CLERK,**
**Suite 301,**
**Sunlight House,**
**Quay Street**
**Manchester M3 3JY (GB)**

EP 0 541 518 B1

**Description**

Among the critical characteristics of paper and other sheet materials which are important to both manufacturers and users is strength. Many different methods for measuring strength have been proposed in the past, but virtually all suffer from a great disadvantage, namely, the tests are destructive and cannot be used "on-line". A number of standardized tests have been devised to provide a basis for specifications by whch paper can be bought and sold, and these tests provide arbitrary but nevertheless useful strength indices for comparing the strengths of various papers. Unfortunately, all are destructive, and none can be used "on-line".

Two problems which make the measurement of strength "on-line" difficult arise from the facts that the strength of paper varies across the width of the sheet being produced, and is also different in the machine direction and the cross sheet direction. Since papermaking is a high speed continuous process, large amounts of paper can easily be produced before strength can be confirmed by a later measurement.

Strength specifications for paper are usually given in terms of an empirical destructive test, the more common of these being a standardized tensile test, the so-called "STFI" compression test, and the "burst pressure" or "Mullen" test.

In the standard tensile test, a strip of paper is held betwen two clamps and loaded in tension at a predetermined rate. The loading at failure is taken to be a measure of the tensile strength of the paper. There are a number of standardized procedures which have been adopted to perform this test, e.g., TAPPI Standard T404os-76 and ASTM Standard D828.

The "STFI" compression test for heavy papers is a standardized test whose procedure has been established by the Swedish Technical Forest Institute, as specified by the identifiers: Scan P46 Column 83. In this test a strip of the paper to be tested is held between a pair of clamps which are moved together at a fixed rate while the compressive force is monitored. "Rupture" occurs when the compressive force passes a peak and begins to drop. The force at "rupture" is taken as the compressive strength of the paper. Other standard specifications for this test are, e.g., TAPPI 7818os-76 and ASTM D1164.

The strengths of papers as measured by the foregoing tests typically have different values depending on whether the test strip is cut in the machine direction or the cross direction.

A "Mullen" or burst pressure test is conducted by clamping a sample of the paper between two circular clamping rings having a specified standard inside diameter, and building up pressure on one side of the paper until the paper bursts (using a rubber diaphram and liquid pressure). The pressure required to burst the paper is known as the "burst pressure" and is the figure often used to specify the required strength. Common burst pressure specifications are TAPPI 403os-76 and ASTM D774.

Needless to say, none of these tests lend themselves to use in connection with the continuous measurement of paper strength. Because of their widespread popularity, however, it is desirable that any method used to measure the strength of paper provides results which correlate with one of the recognized standard tests.

By its nature, a measurement of the strength of paper or other sheet materials is destructive. Fortunately, however, we have found that strength is related to factors which can be measured on a continuous basis by non-destructive means. It is an object of the present invention to provide a system for non-destructively determining the strength of paper and like sheet materials being manufactured, which may be used "on-line", i.e., while the paper or other sheet material is being manufactured.

A system and process for continuously measuring the strength of a moving sheet is disclosed in European Patent Application No. 86400930, which was published after the priority date of the present application. The disclosed apparatus uses the tension, elastic modulus and strength of the sheet as it travels between first and second rolls. The system comprises load measuring means to measure the load of the sheet at a plurality of points between the first and second rolls and to generate signals corresponding to the measured load. Computing means are provided to receive the signals from the load measuring means and to determine the strength of the sheet, measuring the strength of at least one point relative to the strength of at least one other point.

Examples of known apparatus for testing the strength/quality of stationary sheet material without breaking the sheet are disclosed in US Patent No. 2,966,792 and Swiss Patent No. 475679. The latter relates to an apparatus for automatic authenticity testing of bank notes in which the distortion of a bank note under a predetermined applied pressure is measured and used as one of the parameters in measuring the bank note paper quality.

According to the present invention there is provided a sensor for non-destructively determining a physical characteristic of a moving sheet of material comprising:

EP 0 541 518 B1

(a) a contacting means for supporting one side of the moving sheet of material against a deflecting force, said contracting means being disposed around an unsupported region into which the sheet may be deflected;

(b) deflecting means for providing a non-destructive deflecting force for deflecting the sheet into the unsupported region;

(c) a deflection sending device for sensing the distance that the sheet is deflected within the unsupported region and providing a signal in response thereto; and

(d) computing means operatively associated with the sensing device for calculating the physical characteristic of said sheet based upon at least the output of the sensing device.

The major factors which influence the strength of paper, e.g., its basis weight and thickness, also affect the elastic modulus and bending stiffness of the sheet. We have found that these latter factors can be sensed in a sheet under tension in such a way that the actual strength of a sheet can be determined by computation from the output of the sensor. The preferred form of strength sensor used senses a characteristic of the paper which will be referred to as "elastic modulus" since the characteristic is related to the modulus, but is not actually a measure of the elastic modulus of the sheet itself. Quotation marks are used around the term "elastic modulus" to indicate that the function, while related to the elastic modulus is really an empirically derived factor which depends on other characteristics such as bending stiffness also. In any particular paper making set up, changing the speed of the web also affects the strength of the resulting product.

Elastic modulus is difficult, if not impossible to measure directly on a moving web of paper or other sheet material. However, the present application describes a sensor which can sense a physical characteristic of the sheet related to elastic modulus and can provide an output which, when taken together with the other factors such as one or more of the sheet basis weight, thickness, sheet tension and velocity, can be used to determine sheet strength continuously and nondestructively. This sensor can provide a measurement which allows a strength determination to be made which correlates well with the popular "Mullen" test. In the present application, a sensor is described which not only allows a Mullen determination to be made but allows separate determinations of machine and cross direction tensile and compression strengths.

Preferably the deflecting means comprises a free-turning wheel.

Preferably the contacting means comprises a ring defining a circular unsupported region.

The deflecting force is preferably substantially constant.

In a preferred embodiment of the invention the physical characteristic measured is failure strength, in which case the computer means preferably calculates failure strength based on the following equation:

$$S = AZ^H + E$$

where A, E and H = constants;
Z = deflection; and
S = strength.

The "elastic modulus" sensor in the presently preferred embodiment of the present invention uses a sheet (or "web") contact means or support ring which is split into four segments, each occupying approximately 90 degrees of the ring circle. Each segment is supported on a pair of leaf springs and a sensing device such as a load cell so that first means such as a free running wheel acting on the sheet in the centre of the ring will deflect the sheet and result in an output from each of the four load cells, with the output level being dependent in part on sheet characteristics. The four segments are aligned so that two are sensitive to machine direction characteristics. A free running wheel pressing on the sheet at the centre of the support ring provides the force which is transmitted to each load cell.

A computer accepts the outputs of each of the load cells, and may also accept outputs from one or more other sensors which sense basis weight, sheet thickness, velocity, tension and certain correction factors. The computer calculates the sheet strength using these signals in accordance with certain empirical equations which have been developed.

It should be understood that while references are made to a specific standardized test herein, the reference is intended to be exemplary only and not limiting. The present invention can be utilized to provide determinations which correlate well with a wide variety of standardized tensile, compressive and burst pressure tests. Alternatively, the present invention can be utilized to provide an arbitrary index of strength which will allow comparisons of strength as between products independent of any existing standardized system.

A clear understanding of the invention can be had by referring to the following detailed description of the presently preferred embodiments of the invention together with the appended drawings.

3

Brief Description of the Drawings

Figure 1 is a perspective view of the sensing portion of the invented apparatus as installed in a paper making machine.

Figure 2 is a partial cross-sectional side view of the sensor for sensing the "elastic modulus" of the paper.

Figure 3 is a top plan view of the portion of the lower gauge support member in the region of the sensing ring.

Figure 4 is a fragmentary cross-sectional view of the sensing wheel.

Figure 5 is a block diagram of one embodiment of the electronic portion of the invention.

Figure 6 is a partial cross-sectional side view of a second embodiment of an "elastic modulus" sensor.

Figure 7 is a partial cross-sectional side view of a third embodiment of an "elastic modulus" sensor.

Figure 8 is a schematic side view of one means for providing a signal responsive to web tension.

Paper and other sheet-like materials are ordinarily made in a continuous sheet by high speed machines, often several hundred feet in length. The process for making paper involves laying a wet mass of wood pulp onto a moving wire fabric belt, drying the mass, and finally calendering the sheet to give it the desired surface finish. The present invention is most advantageously used to monitor the strength of the paper after the final calendering operation, and before the paper is rolled up on the final reel. A windup motor maintains a constant tension in the sheet between the calender and the reroll reel. Since the strength of the paper produced may vary across the sheet as well as along the sheet, the present invention preferably involves the use of a scanning system whereby the sensors scan cross the width of the paper while the paper is being fed out of the calender and into the reroll system. In this way the variations in strength, side to side, may be determined, as well as the strength of the paper at each section along its length.

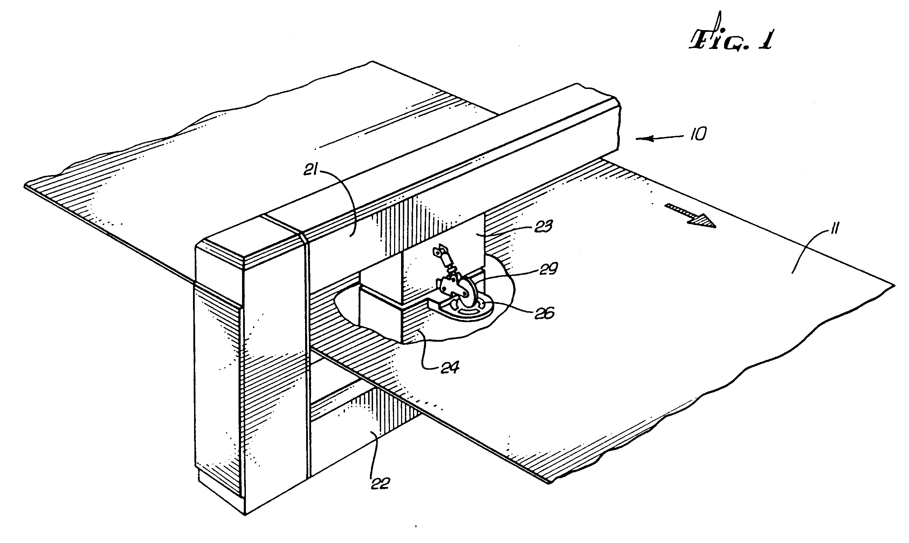

Figure 1 shows a scanning station 10 which, as noted above, is preferably located after the final calender rolls and before the reroll system. A web of paper 11 can be seen passing through the scanning station 10 between two transverse beams 21 and 22, on which are mounted upper and lower gauge support members 23 and 24. The web of paper 11 in Figure 1 is shown with a cut out area so that the relationship between the gauge support members can be seen. The motor within the scanning station is coupled to, and drives the gauge support members 23, 24 back and forth across the width of the paper in a continuous scanning motion, keeping them in alignment at all times.

The gauge support members may carry four or more sets of sensors to provide data that can be used to calculate paper strength. Four factors which may be used are basis weight, thickness, "elastic modulus", and the velocity of the web. Means for determining basis weight, thickness, and paper velocity are all known in the prior art and are therefore not separately shown on the drawings or discussed individually herein. Thickness and paper velocity are relatively simple to measure, and many methods are known in the prior art. Basis weight is a more complex matter, but a suitable method is disclosed in U.S. Patent No. 3,757,122 issued to Bossen et al.

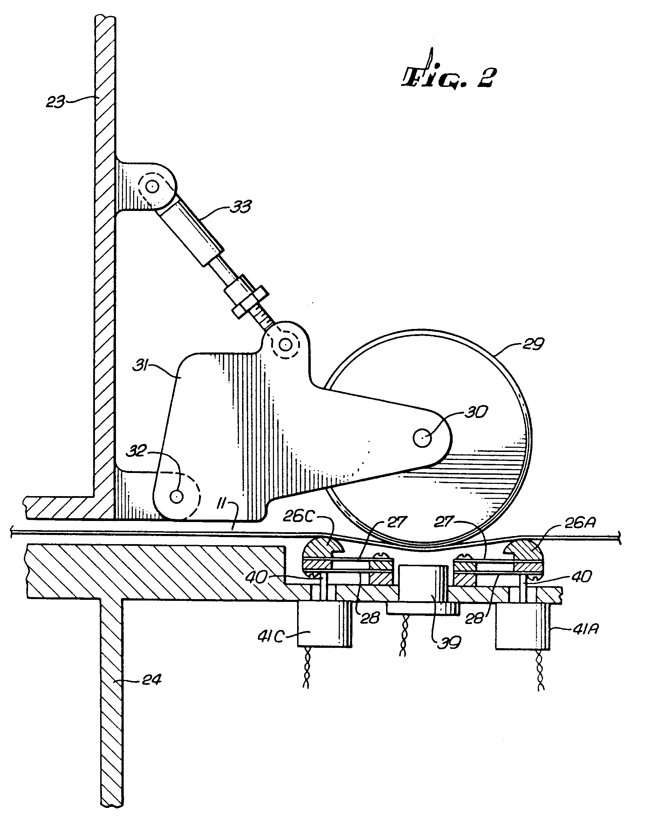

The fourth sensor provides data which relates to the "elastic modulus" of the paper. One such "elastic modulus" sensor used to detect a physical characteristic of the sheet which may be correlated to the sheet or paper strength is shown in Fig. 2. In the particular embodiments of the sensor shown, the characteristic may be sensed by detecting the force required to deflect the sheet a predetermined amount or by detecting the deflection of the sheet when subjected to a predetermined force. Both methods are within the broad scope of the invention.

The "elastic modulus" sensor is carried on gauge supports 23, 24. Fig. 2 shows a partially sectioned side view of this sensor. Lower gauge support 24 supports a segmented horizontal ring 26A-26D whose top surface is preferably aligned with the paper web 11. While a ring such as ring 26 is the preferred method of support, other forms of contacting structures could be used which contact the paper in the vicinity of a central unsupported region.

The strength sensor shown in Fig. 2 provides a stable signal relatively directly related to strength of the sheet material. Such sensor also facilitates a separate strength determination in the cross-direction and machine direction and minimises the need for tension correcting signals in certain circumstances.

Upper gauge support 23 carries a first means such as the pressure wheel assembly which is comprised of bracket 31 and wheel 29. Pin 32 allows the wheel freedom to move up and down while bearings on axle 30 (not shown) permit the wheel to rotate freely. Up and down motion of the wheel 29 is controlled by air cylinder 33. In its extended position, air cylinder 33 positions the lower portion of the periphery of wheel 29 a fixed distance below the top surface of ring 26 and the sheet and the ring 26 are in a cooperative relationship. For purposes of example, and not by way of limitation, if the diameters of wheel 29 and ring 26 are each about 5 inches, a satisfactory position for the lowest point on wheel 29 may be 1/4 inch below the top surface of ring 26. It is also possible to invert the components as shown in Figure 2 so that the ring 26 is above the sheet and the wheel 29 is below the sheet. In a present form of the invention the components are so arranged.

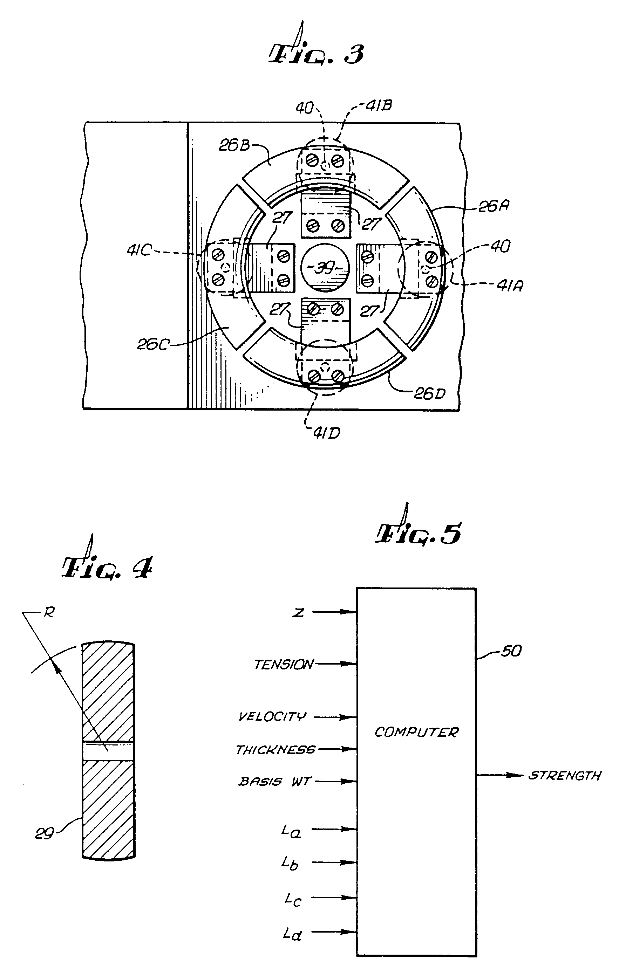

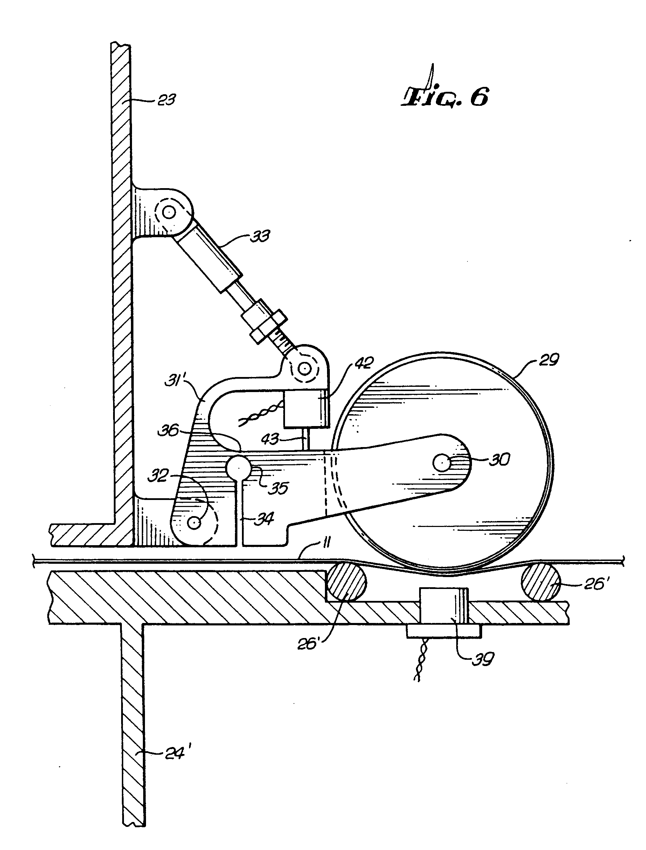

The periphery of wheel 29 is preferably not cylindrical but, rather, is preferably approximately spherical. That is, the radius R as shown in Figure 4 is preferably approximately equal to one-half the wheel diameter. Retraction of air cylinder 33 moves wheel 29 out of the way so that a web of paper 11 can be fed through the scanning station easily upon initial set up. During operation, air cylinder 33 is extended and the sheet is acted upon to move from a normal path to a deflected path in the strength sensor.

It will be understood by those skilled in the art that while a free running wheel for the purpose of deflecting the paper below the top surface of ring 26 is disclosed herein, other structures can be utilized to accomplish the same function.

Figure 3 is a top plan view of the portion of the lower gauge member which supports the segmented ring 26. Referring to both Figures 2 and 3, it can be seen that the ring 26 is comprised of four segments 26A-26D which are each supported by a pair of spaced leaf springs 27 and 28. The leaf springs 27, 28 constrain the segments 26A-26D to move with straight line vertical motion. Linkage pins 40 couple each segment to load cells 41A-41D which provide outputs that are a function of the paper force on the ring segments. The force exerted on each of the segments 26 is a function of several factors including the tension on the sheet, the elastic modulus of the paper, the bending stiffness of the paper, and the physical dimensions of the gauging components. The modulus of elasticity and the bending stiffness of the paper may be different in the machine direction as compared to the cross direction, so that the force applied to load cells 41A and 41C is not necessarily the same as the force applied to load cells 41B and 41D. The difference in tension between the machine direction and the cross-direction also results in a difference in force.

I have found that there is a correlation between the force applied to the load cells 41A and C and the machine direction strength of the paper and also a correlation between the force applied to load cells 41B and D and the cross-direction strength of the paper.

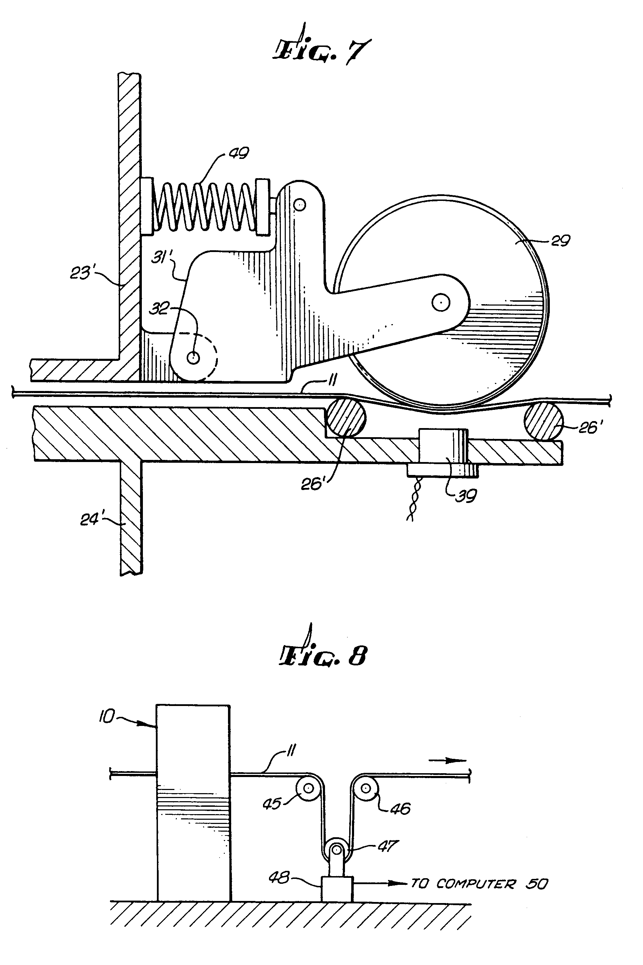

In order to make a strength determination, the outputs of transducers 41A-D are fed to a computer 50 (Fig. 5) which also may receive signals from the other sensors referred to above. The computer 50, by repeated calculations, provides continuous determinations of the strength of the paper as it is being manufactured. Empirical equations have been developed which correlate well with standard machine direction and cross direction strength tests as well as with the non-directional standard burst pressure or "Mullen" test.

The accuracy of the basic equations developed depends upon keeping the alignment of the top surface of ring 26 even with the web of paper 11 as it is being fed into or out of the scanning station 10. If the incoming web 11 is higher or lower than the top surface of ring 26, a correction to the basic equation must be made in order to arrive at an accurate determination of paper strength. I have found that a satisfactory correction can be made by utilizing an additive factor which is a function of the difference between the forces applied to transducers 41A and 41C.

Complete empirical equations for determining directional paper strengths are as follows:

$$S_{md} = A \frac{L_a + L_c}{\overline{L}_a + \overline{L}_c} + (B \times W^E \times T^F) + C \times V^D + G \times (L_a - L_c)^H \quad (1)$$

$$S_{cd} = A \frac{L_b + L_d}{\overline{L}_b + \overline{L}_d} + (B \times W^E \times T^F) + C \times V^D + G \times (L_b - L_d)^H \quad (2)$$

Where
$S_{md}$ is the machine direction strength of the paper;
$S_{cd}$ is the cross direction strength of the paper;
A, B, C, D, E, F, G and H are constants;
$L_a$ through $L_d$ are instantaneous forces applied to transducers 41A through 41D;
$\overline{L}_a$ through $\overline{L}_d$ are the average forces applied to transducers 41A through 41D across the width of the paper;
W is the basis weight of the paper;
T is the thickness of the paper; and

V is the velocity of the paper leaving the calender.

The above equations have been found to correlate well with both the standard tensile and the standard STFI tests for paper strength, both commonly used. The constants A through H vary depending on which test is being simulated. The last additive factor in each of the above equations represents the correction for web misalignment.

A modification of equations (1) and (2) allows the system to be used to determine the "Mullen" strength of the paper:

$$S_{mu} = A \ \frac{L_a + L_b + L_c + L_d}{\overline{L}_a + \overline{L}_b + \overline{L}_c + \overline{L}_d} + (B \times W^E \times T^F) + C \times V^D + G \times (L_a - L_c)^H \quad (3)$$

where

$S_{mu}$ is the "Mullen" strength;

All other factors are the same as in equations (1) and (2).

The above equations have been found to be applicable to a wide variety of papers being manufactured. The constants A, B, C, D, E, and F vary somewhat depending on the particular paper being made, and which test is being simulated, but generally fall within the following ranges (where basis weight is in pounds per 1000 square feet, thickness is in mils, and velocity is in feet per minute):

A from 20 to 22

B from .5 to 5

C from 0 to 0.07

D from 0.5 to 5

E from 1 to 2

F from -1 to +1

The constants G and H must be empirically determined since they are strongly dependent on the particular setup.

It may be noted that the terms of the above equations involving La through Ld are in the form of a ratio with $\overline{L}_a$ through $\overline{L}_d$. This indicates that "elastic modulus" is primarily an idicator of how strength varies across the width of the paper, while the average strength is primarily determined as a function of basis weight, thickness, and web velocity.

Machine rigidity is an important factor in maintaining accuracy of measurement. In particular, it is important that the lowest point on the periphery of wheel 29 be maintained at a constant distance below the top surface of ring 26. If the mechanical rigidity of the structure is such that the spacing cannot be maintained, sensing means can easily be adapted to sense the relative positions of wheel 29 and ring 26, and to apply a correction of the strength equation to account for variations. A conventional sensor for this purpose is shown diagrammatically in Figure 2 identified by the numeral 39. The output of displacement sensor 39, designated as "Z", provides a signal dependent on the deviation in the relative positions of wheel 29 and ring 26 from nominal, and may be used to modify the strength equation. For example, if equation (1) was in use, the first term of the equation would be:

$$f(Z) \ \times \ A \frac{L_a + L_c}{\overline{L}_a + \overline{L}_c}$$

where f(Z) is an empirical function of the output of sensor 39.

We have also found that by properly combining the outputs of load cells 41A-41D it is possible to make a determination, on line, which accurately correlates with the standard "Mullen" test. In order to make such a determination, the outputs of load cells 41A-41D are fed to a computer 50 along with the output of the displacement sensor 39 (Z). The computer 50 calculates the "Mullen" strength. The presently preferred equation which may be used to determine strength is:

6

$$S_{mu} = A \left( \frac{L_a + L_c}{(C+Z) \ Te+F)} \right)^H + B \left( \frac{L_b + L_d}{(D+Z) \ Te+G)} \right)^J + E \qquad (4)$$

where $S_{mu}$ is the "Mullen" strength of the paper;

A, B, C, D, E, F, G, H and J are constants;

$L_a$, $L_b$, $L_c$ and $L_d$ are the output signals from load sensors 41A through 41D, respectively;

Te is a number representative of the tension in the web; and

Z is the output of displacement sensor 39.

If the gauge support members are held with adequate rigidity so that there is a sufficiently small relative motion between members 23 and 24, sensor 39 would not be needed, and the terms C + Z and D + Z could be replaced with constants. Similarly, if tension in the web does not vary significantly, the terms Te + F and Te + G could be replaced by constants. Thus, if in a particular installation the variations due to lack of rigidity and variations in tension are low enough, equation (4) could have the following form:

$$S_{mu} = A \left( \frac{L_a + L_c}{C} \right)^H + B \left( \frac{L_b + L_d}{D} \right)^J + E \qquad (4a)$$

The values of the constants in equations (4) and (4a) will, of course, depend on the particular installation and the circumstances. They are empirically determined, and will, in general, be different as used in equation (4) as compared to (4a). The use of exponents H and J is not intended to indicate a value necessarily different from 1. In some cases, depending on the installation and circumstances, one or both of these exponents may be 1. In addition, the constants may be found to be truly constant (within the desired accuracy of measurement) only if the process variations are relatively small, i.e., within the ranges normally occurring durig the manufacture of a single grade of paper. Unusually large variations in one or more of the process variables may require that one or more of the equation "constants" be adjusted to account for the variation.

There are many ways known in the art for providing a signal to computer 50 which is a function of the tension in the web. One such system is shown schematically in Figure 8. As shown, the web 11 is threaded around three rollers 45, 46 and 47. Rollers 45 and 46 are fixed relative to the paper making machine and roller 47 is restrained from moving in a vertical direction by force transducer 48. The output of force transducer 48 will be a function of the tension of web 11 can be used to provide the quantity Te.

While the above equations provides strength values which correlate with the "Mullen" strength, as noted, it will be evident to those skilled in the art that it is possible to obtain other numbers which are also representative of strength using the strength sensor outputs in connection with different equations, even though such other numbers may not correlate with "Mullen".

For example, if a determination of machine direction and/or cross direction tensile strength is desired, equations in the following forms could be used:

$$S_{md} = A \left( \frac{L_a + L_c}{(C+Z) \ (Te+F)} \right)^H + E \qquad (5)$$

and

$$S_{cd} = B \left( \frac{L_b + L_d}{(D+Z) \ (Te+G)} \right)^J + E \qquad (6)$$

where $S_{md}$ and $S_{cd}$ are the machine direction and cross direction tensile strengths, respectively; and the other terms are as previously defined.

By proper choice of the constants, equations (5) and (6) can provide determinations which correlate well with standard tensile tests. While the same letters (A, B, C, etc.) are used in equations 4, 4a, 5 and 6 to represent constants, it should not be assumed that the constants as used in these equations necessarily have the same values. As noted above, the actual values of the constants will depend on the particular installation and circumstances. The comments above concerning modification of equation (4) on account of the effects of rigidity and tension are also applicable to equations (5) and (6) and similar simplified equations can be used if rigidity and/or tension variations are small enough. That is, the terms in the denominator of the first term of the equations could be replaced by a constant.

A partial cross sectional view of a second type of sensor suitable for use in the invented system is shown in Figure 6. The main difference between the sensor of Figure 6 and that shown in Figures 2 and 3 is that only a single load cell 42 is used, which provides an output representative of the force between wheel 29 and sheet 11 rather than the forces between sheet 11 and each of the segments of ring 26. In the embodiment of Figure 6, since no instrumentation of the ring is required, an unsegmented ring 26' may be used solidly mounted to gauge carrier 24'. Bracket 31' is similar to bracket 31 except that it is cut out to receive load cell 42 and is made deformable by slot 34 and bore 35 which leaves a thin flexible portion 36. The force acting between the sheet 11 and wheel 29 is thus sensed by load cell 42 through linkage pin 43.

Using the sensor of Figure 6, a strength determination which can be correlated with the "Mullen" test can be obtained using the following equation:

$$S_{mu} = A \left( \frac{L_e}{(C+Z) \ (Te+F)} \right)^H + E \qquad (7)$$

where $L_e$ is the load on load cell 42; and the other terms are as previously defined.

An equation similar to Equation 7 can also be used in conjunction with the sensor of Figure 2, where $L_e$ is the load on one of the transducers 41 or an average or sum of the outputs of two or more.

If the machine rigidity and/or variations in tension are low enough for the accuracy required, the terms $(C + Z)$ and/or $(Te + F)$ can be replaced with constants, resulting in the following equation:

$$S = A \left( \frac{L}{C} \right)^H + E \qquad (7a)$$

Where

S is a measure of the strength of the sheet;

L is the load on the load cell(s) used; and

A, C, E and H are constants.

For optumum accuracy, under some circumstances it has been found desirable to modify the strength values as determined by equations 4-7. In particular, variations in basis weight and sheet density can be the source of inaccuracy in the results obtained and one form of modified equation which may be used to obtain more accurate results is:

$$S_{corr} = K(\ln(BW) + M(d-do)^N + S_{meas} \qquad (8)$$

Where

$S_{meas}$ is the strength as determined from equations 4-7

$S_{corr}$ is the corrected strength value;

ln(BW) is the natural logarithm of the basis weight of the sheet;

d and do are the actual and nominal densities of the sheet; and

K, M, and N are constants.

The foregoing equations relate to embodiments of the invention wherein a load cell type of sensor is employed. As mentioned it is within the broad scope of the invention to employ other related sensors that provide a signal representative of a physical characteristic which may be correlated to physical strength of the sheet materials. Detecting the deflection of the sheet material loaded by a relatively constant force (e.g. the weight of wheel 29) will provide such a signal. In the previously described embodiments of the invention, the sheet material is deformed a predetermined amount and the forces involved are sensed. In the sensor arrangement of this alternative system, a relatively constant force is applied to the sheet material and the resulting deflection of the sheet is sensed and processed to determine strength. For example, the sensor could be substantially similar to that shown in Figure 6 with the component 42 omitted. The weight of the wheel (as is or with added weight) could provide a constant force that deflects the sheet material. The resulting deflection is measured by the sensor 39. Other force applying devices could be utilized such as springs or electronically controlled actuators. A diagrammatic side view of a sensor according to the embodiment just described is shown in Figure 7. Spring 49 provides a downward force on the sheet in addition to that due to the weight of wheel 29. The strength of the sheet material, assuming a relatively constant sheet tension, may be determined by an equation of the form of:

$$S = AZ^H + E \qquad\qquad (8)$$

where A, E and H are constants and Z is the relevant deflection. With a deflection sensor employed, it is possible to employ a sensor with ring 26 or without such ring. Corrections to account for variations in tension and machine rigidity can be made as discussed in connection with the first sensor embodiment described.

It is also within the scope of the invention to employ both a force sensor and a deflection sensor. The resulting strength determinations could be combined or averaged or the signals resulting from the sensors could be employed in a modified equation.

What has been disclosed is systems and methods for determining the strength of a sheet of material, such, for example, paper, which is nondestructive and which can be used "on-line" to assure that the paper or other sheet material being manufactured meets the required strength specifications.

A presently preferred embodiment of the invention includes a segmented ring with four segments and four load cells, but that is not the only possible number of sheet supporting areas. It is possible to get meaningful information from even a single load cell coupled to one of the segments. As can be easily seen, many combinations are possible within the teachings of the above disclosure, therefore the scope of the invention should be limited only by the following claims.

**Claims**

1. A sensor for non-destructively determining a physical characteristic of a moving sheet of material (11) comprising:

   (a) a contacting means (26A, 26B, 26C, 26D, 26') for supporting one side of the moving sheet of material (11) against a deflecting force, said contacting means (26A, 26B, 26C, 26D, 26') being disposed around an unsupported region into which the sheet (11) may be deflected;

   b) deflecting means (29) for providing a non-destructive deflecting force for deflecting the sheet (11) into the unsupported region;

   (c) a deflection sensing device (39) for sensing the distance that the sheet (11) is deflected within the unsupported region and providing a signal in response thereto; and

   (d) computing means (5) operatively associated with the sensing device (39) for calculating the physical characteristic of said sheet (11) based upon at least the output of the sensing device (39).

2. A sensor as claimed in claim 1, wherein the deflecting means (29) comprises a free-turning wheel.

3. A sensor as claimed in claim 1 or 2, wherein the contacting means (26A, 26B, 26C, 26D, 26') comprises a ring defining a circular unsupported region.

4. A sensor as claimed in any of claims 1 to 3, wherein the deflecting force is substantially constant.

5. A sensor as claimed in any of claims 1 to 4, wherein the sheet material (11) is paper.

6. A sensor as claimed in any of claims 1 to 5, wherein the physical characteristic is failure strength.

7. A sensor as claimed in claim 6, wherein the computer means calculates failure strength based on the following equation:

$$S = AZ^H + E$$

where A, E and H = constants;
Z = deflection; and
S = strength.

**Patentansprüche**

1. Fühler zum zerstörungsfreien Bestimmen einer physikalischen Eigenschaft eines in Bewegung befindlichen Materialbogens (11), der folgendes umfaßt:

   (a) ein Berührungsmittel (26A, 26B, 26C, 26D, 26') zum Stützen einer Seite des in Bewegung befindlichen Materialbogens (11) gegen eine Ablenkkraft, wobei das genannte Berührungsmittel (26A, 26B, 26C, 26D, 26') um einen ungestützten Bereich herum angeordnet ist, in den der Materialbogen (11) abgelenkt werden kann;

   (b) Ablenkmittel (29) zum Vorsehen einer zerstörungsfreien Ablenkkraft zum Ablenken des Bogens (11) in den ungestützten Bereich;

   (c) eine Ablenkungsfühlvorrichtung (39) zum Ertasten der Entfernung, um die der Bogen (11) innerhalb des ungestützten Bereichs abgelenkt wird, und zum Vorsehen eines darauf ansprechenden Signals; und

   (d) eine der Fühlvorrichtung (39) funktionsmäßig zugeordnete Recheneinrichtung (5) zum Errechnen der Physikalischen Eigenschaft des genannten Bogens (11) auf der Grundlage von mindestens der Ausgabe der Fühlvorrichtung (39).

2. Fühler nach Anspruch 1, bei dem das Ablenkmittel (29) ein sich frei drehendes Rad aufweist.

3. Fühler nach Anspruch 1 oder 2, bei dem das Berührungsmittel (26A, 26B, 26C, 26D, 26') einen Ring aufweist, der einen kreisförmigen ungestützten Bereich definiert.

4. Fühler nach einem der Ansprüche 1 bis 3, bei dem die Ablenkkraft im wesentlichen konstant ist.

5. Fühler nach einem der Ansprüche 1 bis 4, bei dem das Bogenmaterial (11) Papier ist.

6. Fühler nach einem der Ansprüche 1 bis 5, bei dem die physikalische Eigenschaft die Ausfallfestigkeit ist.

7. Fühler nach Anspruch 6, bei dem die Computereinrichtung die Ausfallfestigkeit auf der Grundlage der folgenden Gleichung berechnet:

$$S = AZ^H + E,$$

wobei A, E und H = Konstanten;
Z = Ablenkung; und
S = Festigkeit sind.

**Revendications**

1. Capteur pour déterminer de manière non-destructive une caractéristique physique d'une feuille en mouvement de matériau (11) comprenant :

   (a) un moyen de contact (26A, 26B, 26C, 26D, 26') servant à soutenir un côté de la feuille en mouvement de matériau (11) à l'encontre d'une force de déflexion, ledit moyen de contact (26A, 26B, 26C, 26D, 26') étant disposé autour d'une région non soutenue dans laquelle la feuille (11) peut être défléchie ;

   (b) un moyen de déflexion (29) servant à fournir une force de déflexion non-destructive servant à défléchir la feuille (11) dans la région non soutenue ;

(c) un dispositif de détection de déflexion (39) servant à capter la distance sur laquelle la feuille (11) est défléchie au sein de la région non soutenue et fournissant un signal en réponse à ceci ; et
(d) un moyen de calcul (5) associé activement au dispositif de détection (39) servant à calculer la caractéristique physique de ladite feuille (11) sur la base d'au moins la sortie du dispositif de détection (39).

2. Capteur selon la revendication 1, dans lequel le moyen de déflexion (29) comprend une roue tournant librement.

3. Capteur selon la revendication 1 ou 2, dans lequel le moyen de contact (26A, 26B, 26C, 26D, 26') comprend un anneau définissant une région circulaire non soutenue.

4. Capteur selon l'une quelconque des revendications 1 à 3, dans lequel la force de déflexion est sensiblement constante.

5. Capteur selon l'une quelconque des revendications 1 à 4, dans lequel le matériau de feuille (11) est du papier.

6. Capteur selon l'une quelconque des revendications 1 à 5, dans lequel la caractéristique physique est la résistance à la défaillance.

7. Capteur selon la revendication 6, dans lequel le moyen de calcul calcule la résistance à la défaillance sur la base de l'équation suivante :

$$S = AZ^H + E$$

dans laquelle A, E et H = constantes :
Z = déflexion ; et
S = résistance.

Fig. 1

Fig. 2

## Fig. 3

40  41B

26B

27  27

41C  40

~39~

41A

27  27

26C

26D

41D

## Fig. 4

R

29

## Fig.5

Z →

TENSION →

VELOCITY →

THICKNESS →

BASIS WT →

COMPUTER

50

→ STRENGTH

$L_a$ →

$L_b$ →

$L_c$ →

$L_d$ →

14

Fig. 6

# Fig. 7

# Fig. 8

TO COMPUTER 50